# EUROPÄISCHE PATENTSCHRIFT

(11) **EP 2 796 206 B1**
(45) Veröffentlichungstag und Bekanntmachung des Hinweises auf die Patenterteilung: **21.12.2016**
(21) Anmeldenummer: 13165629.0
(22) Anmeldetag: 26.04.2013
(51) Int. Cl.: B05B 11/00, A61M 15/00, A61M 15/08, B65D 83/22

(54) **Austragkopf für einen Behälter und Verfahren zur Befestigung eines Austragkopfs an einem Behälter**
Dispensing head for a container and method for fixing a dispensing head on a container
Tête de distribution associée à un récipient et procédé de fixation d'une tête de distribution sur un récipient

(43) Veröffentlichungstag der Anmeldung: 29.10.2014
(73) Patentinhaber: Aptar Radolfzell GmbH, 78315 Radolfzell (DE)
(72) Erfinder: Greiner-Perth, Jürgen, 78244 Gottmadingen (DE)
(74) Vertreter: Patentanwaltskanzlei Cartagena

(56) Entgegenhaltungen:
- DE-A1- 1 475 179
- FR-A1- 2 527 562
- FR-A1- 2 635 085
- FR-A1- 2 946 962
- GB-A- 1 109 829
- US-A- 3 403 823

## Beschreibung

Die Erfindung betrifft einen Austragkopf zum Austragen eines flüssigen oder pastösen Mediums, welcher an einem das Medium bevorratenden Behälter befestigbar ist, umfassend ein Zwischenelement mit einer Aufnahmeöffnung für den Behälter und eine in Axialrichtung des Zwischenelements verschieblich mit dem Zwischenelement gekoppelte Betätigungshandhabe. Die Erfindung betrifft weiter einen Spender umfassend einen Austragkopf und einen Behälter und ein Verfahren zur Befestigung eines Austragkopfs an einem Behälter.

Das Zwischenelement wird mit dem Behälter verbunden. Die Betätigungshandhabe ist relativ zu dem Zwischenelement und damit relativ zu dem Behälter zum Austragen eines Mediums bewegbar.

Aus FR 2 635 085 A1, US 3,403,823, GB 1,109,929, DE 1 475 179 A1 und FR 2 527 562 A1 sind jeweils Austragköpfe für Spender mit einem Manipulationsschutz bekannt, wobei der Manipulationsschutz als mit einer Sollbruchstelle realisierte starre Verbindung zwischen einer Betätigungshandhabe und einem Gehäuse (Zwischenelement) realisiert ist. Dabei ist vorgesehen, dass bei einem ersten Gebrauch die starre Verbindung manuell durch einen Nutzer gelöst wird.

Derartige Austragköpfe sind sowohl für kosmetische Produkte als auch für Medikamente einsetzbar. Der Austragkopf wird üblicherweise nach einem Befüllen des Behälters durch einen Abfüller an dem Behälter angebracht. Dabei sind verschiedene Befestigungsverfahren, wie Verschrauben, Verkleben oder dergleichen bekannt.

Sofern der Behälter ein Medikament bevorratet ist es wünschenswert, dass eine Verbindung zwischen dem Behälter und dem Austragkopf nur durch Aufbringen hoher Kräfte und/oder, beispielsweise für eine Kindersicherung, nur durch komplexe Bewegungsmuster möglich ist. Es ist daher bekannt, an dem Behälter und/oder dem Austragkopf eine Rastgeometrie vorzusehen, wobei der Austragkopf auf den Behälter aufgeschnappt und/oder aufgeprellt wird. Als Aufprellen wird dabei im Zusammenhang mit der Anmeldung eine Herstellung einer Rastverbindung durch impulsartig aufgebrachte Kräfte bezeichnet. Im Unterschied hierzu erfolgt ein Aufschnappen durch eine Relativbewegung der zu verbindenden Bauteile, wobei Montagekräfte über einen von dem jeweiligen Montagevorgang abhängigen, längeren Zeitraum einwirken.

Aus der EP 2 388 075 A1, die einen Austragkopf gemäß dem Oberbegriff des Anspruchs 1 offenbart, ist es bekannt, einen Sprühkopf auf einen Behälter aufzuprellen. Dabei wird eine Schutzkappe über dem Sprühkopf angeordnet, wobei Kräfte zum Aufprellen an der Schutzkappe aufgebracht und mittels der Schutzkappe an die Befestigungsgeometrie übertragen werden. Mittels einer derartigen Schutzkappe soll es möglich sein, den Sprühkopf auf den Behälter aufzuprellen, ohne die Pumpe im übrigen zu belasten.

FR 2 946 962 A1 beschreibt einen Spender mit einem Sprühkopf, wobei der Sprühkopf vor einer Montage in einem als Hülse gestalteten Zwischenelement aufgenommen ist und aus dieser Hülse während der Montage "verdrängt wird". Für die Montage an einem Behälter wird eine Kraft über die Hülse eingeleitet.

### Aufgabe und Lösung

Aufgabe der Erfindung ist es, einen Austragkopf zu schaffen, welcher sicher und einfach an einem Behälter befestigbar ist. Eine weitere Aufgabe der Erfindung ist es, ein Verfahren zur sicheren und einfachen Befestigung eines Austragkopfs an einem Behälter zu schaffen.

Diese Aufgabe wird durch die Gegenstände mit den Merkmalen der Ansprüche 1, 4 und 6 gelöst. Weitere Vorteile der Erfindung ergeben sich aus den Unteransprüchen.

Gemäß einem ersten Aspekt der Erfindung wird ein Austragkopf zum Austragen eines flüssigen oder pastösen Mediums geschaffen, der an einem das Medium bevorratenden Behälter befestigbar ist und der ein Zwischenelement mit einer Aufnahmeöffnung für den Behälter und eine verschieblich mit dem Zwischenelement gekoppelte Betätigungshandhabe umfasst, wobei das Zwischenelement und/oder die Betätigungshandhabe mindestens ein lösbares Verbindungselement aufweisen, das eine Haltekraft zwischen dem Zwischenelement und der Betätigungshandhabe bewirkt, welche einer Relativbewegung zwischen dem Zwischenelement und der Betätigungshandhabe in Betätigungsrichtung entgegensteht, wobei die Aufnahmeöffnung eine Rastgeometrie zur Befestigung an dem Behälter aufweist, und wobei eine zum Aufschnappen und/oder Aufprellen des Zwischenelements auf den Behälter aufzubringende Kraft in Betätigungsrichtung kleiner ist als die durch das mindestens eine Verbindungselement aufgebrachte Haltekraft zwischen dem Zwischenelement und der Betätigungshandhabe in Betätigungsrichtung.

Das Zwischenelement wird während der Montage mit dem Behälter verbunden. Bei dem "Zwischenelement" handelt es sich in einer Ausgestaltung um ein im Wesentlichen rohrförmiges Bauteil, welches auch als Hülse bezeichnet wird. Im Zusammenhang mit der Anmeldung ist die Gestaltung des Zwischenelements jedoch nicht auf die Gestaltung als Hülse beschränkt. Das Zwischenelement kann eine durch den Fachmann geeignet wählbare Geometrie aufweisen, welche eine verschiebliche Kopplung mit der Betätigungshandhabe ermöglicht.

Das Zwischenelement und die Betätigungshandhabe sind in dem fixierten Zustand an dem Behälter fixierbar, wobei eine Kraft über die Betätigungshandhabe eingebracht wird.

Ein Befüllen des Behälters und ein Anbringen des Austragkopfs an dem Behälter erfolgt üblicherweise bei einem Abfüller. Mittels des Verbindungselements ist in einem Anlieferungszustand an den Abfüller eine Fixierung der im Gebrauch verschieblich mit dem Zwischenelement gekoppelten Betätigungshandhabe möglich. Dadurch wird eine undefinierte Lage zwischen der Betätigungshandhabe und dem Zwischenelement, welche die Montage des Austragkopfs erschwert, vermieden. Das Verbindungselement ist während oder nach einer Montage lösbar.

Nach einer Montage des Austragkopfs auf den Behälter ist somit eine Relativbeweglichkeit zwischen dem Zwischenelement und der verschieblich mit dem Zwischenelement gekoppelten Betätigungshandhabe, und folglich auch zwischen der Betätigungshandhabe und dem Behälter, an welchem das Zwischenelement befestigt ist, nutzbar, um einen Austrag des in dem Behälter bevorrateten Mediums zu bewirken. Die Betätigungshandhabe weist zu diesem Zweck in einer Ausgestaltung geeignet Angriffsflächen, wie Fingerauflagen oder dergleichen auf. In anderen Ausgestaltungen wird für einen Austrag des Mediums eine Kraft auf den Behälter aufgebracht, durch welche eine Relativbewegung zwischen dem Behälter und der Betätigungshandhabe bewirkt wird.

Die Rastgeometrie umfasst in einer Ausgestaltung einen Rasthaken, welcher einen Rastvorsprung, eine Rastnut, einen Rast-Hinterschnitt oder dergleichen an einem Gegenstück hintergreift. In vorteilhaften Gestaltungen weist die Aufnahmeöffnung ein einen Rasthaken auf. Es sind jedoch auch Gestaltungen denkbar, bei welchen an dem Behälter ein Rasthaken vorgesehen ist.

Das Medium ist in dem Behälter in einer Ausgestaltung unter Druck bevorratet, wobei aufgrund der Relativbewegung ein Ventil für einen Austrag öffnet. In einer anderen Ausgestaltung ist eine Pumpvorrichtung vorgesehen, welche durch die Relativbewegung zwischen der Betätigungshandhabe und dem Behälter betätigt wird. Die Pumpvorrichtung ist dabei in einer Ausgestaltung an dem Behälter vor einer Montage des Austragkopfs vormontiert.

In einer Ausgestaltung dient die Betätigungshandhabe auch als Gehäuse des Austragkopfs, welches nach der Montage beispielsweise von dem Behälter abragende Elemente der Pumpeinrichtung einhaust. Alternativ oder zusätzlich sind in anderen Ausgestaltungen die Elemente der Pumpeinrichtung in dem Zwischenelement eingehaust.

In vorteilhaften Ausgestaltungen weist die Betätigungshandhabe eine Austragöffnung auf, welche mittels eines Austragkanals mit einer Auslassöffnung des Behälters und/oder einer in dem Behälter angeordneten Pumpeinrichtung kommuniziert ist. In anderen Ausgestaltungen ist eine Austragöffnung an dem Zwischenelement vorgesehen.

Das mindestens eine Verbindungselement ist in einer Ausgestaltung derart gestaltet, dass es bei einer Montage zerstört wird und damit nach einer Montage einer Relativbewegung zwischen dem Zwischenelement und der Betätigungshandhabe nicht entgegensteht.

In anderen Ausgestaltungen wird das mindestens eine Verbindungselement entriegelt oder gelöst, wobei das Zwischenelement und die Betätigungshandhabe nach Lösen des mindestens einen Verbindungselements innerhalb eines Verschiebewegs verschieblich miteinander gekoppelt sind. Um zu verhindern, dass das gelöste Verbindungselement eine Relativbewegung zum Austragen stört, ist in vorteilhaften Ausgestaltungen das Verbindungselement außerhalb des Verschiebewegs angeordnet ist. Die Umkehrpunkte werden im Zusammenhang mit der Anmeldung als oberer und unterer Totpunkt bezeichnet, wobei bei Erreichen des oberen Totpunkts die Bauteile maximal voneinander entfernt sind und bei Erreichen des unteren Totpunkts die Bauteile maximal ineinander geschoben sind. Das Verbindungselement liegt vorzugsweise in einem Bereich hinter dem oberen Totpunkt, sodass die Bauteile bei der Montage über den Bereich des Verschiebewegs voneinander entfernt angeordnet sind.

Wie oben beschrieben, liegt das Verbindungselement vorzugsweise in einem Bereich hinter dem oberen Totpunkt, sodass die Bauteile bei der Montage über den Bereich des Verschiebewegs voneinander entfernt angeordnet sind. Dadurch wird eine Kollision der Betätigungshandhabe mit Elementen einer Pumpeinheit zu Beginn einer Montage verhindert.

In einer weiteren Ausgestaltung ist an der Aufnahmeöffnung ein Anschlag vorgesehen ist, mittels welchem eine Bewegung des Zwischenelements relativ zu dem Behälter beim Aufschnappen und/oder Aufprellen begrenzt ist. Ein Abstand zwischen dem mindestens einen Anschlag und einer Anlagefläche der Rastgeometrie bestimmt somit einen Befestigungsbereich für den Behälter. Je nach Höhe des Befestigungsbereichs ist nach dem Aufschnappen und/oder Aufprellen das Zwischenelement unter Spiel und/oder klemmend mit dem Behälter verbunden.

Gemäß einem zweiten Aspekt wird ein Spender für ein flüssiges oder pastöses Medium geschaffen, mit einem das Medium bevorratenden Behälter und einem an dem Behälter befestigten Austragkopf, umfassend ein Zwischenelement mit einer Aufnahmeöffnung für den Behälter und eine in einer Betätigungsrichtung verschieblich mit dem Zwischenelement gekoppelte Betätigungshandhabe, wobei das Zwischenelement und/oder die Betätigungshandhabe mindestens ein lösbares Verbindungselement aufweisen, das eine Haltekraft zwischen dem Zwischenelement und der Betätigungshandhabe bewirkt, welche einer Relativbewegung zwischen dem Zwischenelement und der Betätigungshandhabe in Betätigungsrichtung entgegensteht.

Der Behälter weist eine zu der Aufnahmeöffnung komplementäre Befestigungsgeometrie auf. In einer Ausgestaltung ist an einer Öffnung des Behälters eine Krimphülse angeordnet. Der Behälter ist mittels der Krimphülse sicher verschlossen. Die Krimphülse lagert in vorteilhaften Ausgestaltungen auch ein Ventil und/oder einem Pumpeinheit zum Austragen des Mediums. Die Krimphülse ist in vorteilhaften Ausgestaltungen derart gestaltet, dass sie mindestens zwei plane Flächen aufweist, an welchen eine Rastgeometrie und ein Anschlag der Aufnahmeöffnung angreifen.

Gemäß einem dritten Aspekt wird ein Verfahren zum Befestigen eines Austragkopfs zum Austragen eines flüssigen oder pastösen Mediums an einem das Medium bevorratenden Behälter geschaffen, wobei der Austragkopf ein Zwischenelement mit einer Aufnahmeöffnung für den Behälter und eine in eine Betätigungsrichtung verschieblich mit dem Zwischenelement gekoppelte Betätigungshandhabe umfasst und wobei die Aufnahmeöffnung eine Rastgeometrie zur Befestigung an dem Behälter aufweist, umfassend den Schritt Aufschnappen und/oder Aufprellen des Zwischenelements auf den Behälter durch Bewegung der mit dem Zwischenelement gekoppelten Betätigungshandhabe in Betätigungsrichtung, wobei beim Aufschnappen und/oder Aufprellen des Zwischenelements auf den Behälter eine Relativbewegung zwischen dem Zwischenelement und der Betätigungshandhabe in Betätigungsrichtung durch mindestens ein Verbindungselement, das eine Haltekraft in Betätigungsrichtung bewirkt, gesperrt wird, nach einem Aufschnappen und/oder Aufprellen des Zwischenelements auf den Behälter das mindestens eine Verbindungselement gelöst wird, und das Zwischenelement auf den Behälter aufgeschnappt und/oder aufgeprellt wird, bevor auf das mindestens eine Verbindungselement eine Kraft wirkt, welche die durch das mindestens eine Verbindungselement aufgebrachte Haltekraft zwischen dem Zwischenelement und der Betätigungshandhabe in Betätigungsrichtung übersteigt.

Dank der Fixierung wird eine undefinierte Lage zwischen dem Zwischenelement und der Betätigungseinrichtung verhindert. Die Fixierung erfolgt dabei vorzugsweise derart, dass das Zwischenelement und die Betätigungseinrichtung nicht in einem unteren Totpunkt des Verschiebewegs angeordnet sind. Vorzugsweise sind das Zwischenelement und die Betätigungseinrichtung über einen oberen Totpunkt eines im Gebrauch vorgesehenen Verschiebewegs hinaus relativ zueinander verschoben und fixiert. Das Zwischenelement ist durch Bewegung in Betätigungsrichtung auf den Behälter aufschnappbar und/oder aufprellbar. Die Aufnahmeöffnung und/oder der Behälter weisen zu diesem Zweck eine Rastgeometrie auf. Durch geeignete Gestaltung der Rastgeometrie und des mindestens einen Verbindungselements ist dabei vorgesehen, dass das Zwischenelement auf den Behälter aufgeschnappt und/oder aufgeprellt wird, bevor auf das mindestens eine Verbindungselement eine Kraft wirkt, welche die durch das mindestens eine Verbindungselement aufgebrachte Haltekraft zwischen dem Zwischenelement und der Betätigungshandhabe in Betätigungsrichtung übersteigt. Dadurch ist sichergestellt, dass während der Montage durch Bewegung in Betätigungsrichtung die Betätigungshandhabe an dem Zwischenelement fixiert bleibt.

In einer weiteren Ausgestaltung ist vorgesehen, dass eine Bewegung des Zwischenelement relativ zu dem Behälter beim Aufschnappen und/oder Aufprellen durch mindestens einen an der Aufnahmeöffnung angeordneten Anschlag begrenzt wird, wobei durch eine weitere Bewegung der Betätigungshandhabe in Betätigungsrichtung auf das mindestens eine Verbindungselement in Betätigungsrichtung eine die Haltekraft übersteigende Kraft aufgebracht wird, sodass das mindestens eine Verbindungselement löst. Wie oben beschrieben, sind vorzugsweise das Zwischenelement und die Betätigungseinrichtung über einen oberen Totpunkt eines im Gebrauch vorgesehenen Verschiebewegs hinaus relativ zueinander verschoben und fixiert. Der Anschlag ist vorzugsweise so positioniert, dass bei Erreichen des Anschlags mit Ausnahme der Befestigungsgeometrie noch kein Kontakt zwischen den Bauteilen des Austragkopfs und den Bauteilen des Behälters stattfindet. Nach Fixierung des Austragkopfs an dem Behälter wird somit noch ein versehentliches Austragen des Mediums verhindert. Erst durch Aufbringen einer weiteren Montagekraft wird ein Lösen des Verbindungselements bewirkt, wobei auch die Bauteile des Austragkopfs, wie ein Austragkanal, und des Behälters, insbesondere einer Pumpeinheit, miteinander verbunden werden.

In einer weiteren Ausgestaltung ist vorgesehen, dass an einer Öffnung des Behälters eine Ventileinheit und/oder eine Pumpeinheit mittels einer Krimphülse vormontiert wird.

### Kurzbeschreibung der Zeichnungen

Weitere Aspekte und Vorteile der Erfindung ergeben sich außer aus den Ansprüchen auch aus der nachfolgenden Beschreibung bevorzugter Ausführungsbeispiele der Erfindung. Dabei zeigen:
- Fig. 1:: schematisch einen Austragkopf 1 für einen Spender;
- Fig. 2:: schematisch einen Behälter 2 für einen Spender;
- Fig. 3 bis 5:: schematisch eine Montage des Austragkopfs 1 gemäß Fig. 1 an einem Behälter 2 gemäß Fig. 2; und
- Fig. 6 und 7:: schematisch eine Betätigung des montierten Austragkopfs 1 gemäß Fig. 1 zum Austragen eines Mediums.

### Detaillierte Beschreibung eines Ausführungsbeispiels

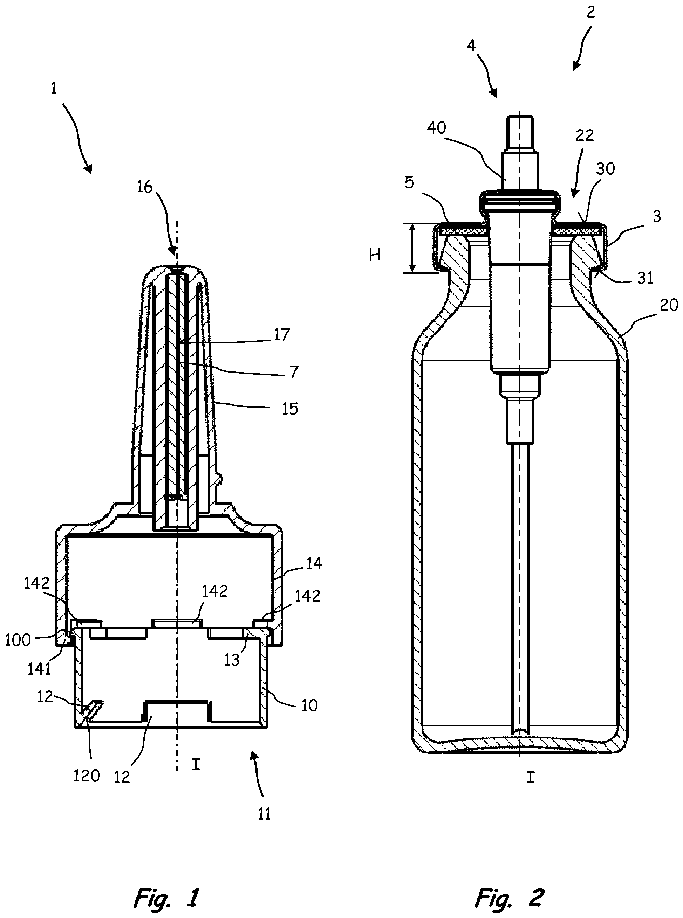

Fig. 1 zeigt schematisch einen Austragkopf 1 für einen Spender zum Austragen von flüssigen oder pastösen Medien. Der Austragkopf ist beispielsweise an einem Behälter 2 befestigbar, wie er in Fig. 2 dargestellt ist.

Der in Fig. 2 dargestellte Behälter 2 umfasst einen Grundkörper 20 zum bevorraten eines Mediums mit einer Öffnung 22, welche durch eine Krimphülse 3 verschlossen ist. Mittels der Krimphülse 3 ist eine Pumpeinheit 4 gelagert. Die Pumpeinheit 4 umfasst einen Kolben 40, welcher zum Austragen des bevorrateten Mediums entgegen der Kraft einer nicht sichtbaren Rückstellfeder in eine Betätigungsrichtung I verschiebbar ist. Der dargestellte Kolben 40 ragt von dem Grundkörper 20 ab. Die Gestaltung der Pumpeinheit 4 ist lediglich beispielhaft.

Zwischen dem Grundkörper 20 und der Krimphülse 3 ist ein Dichtelement 5 angeordnet. Der Behälter 2 bildet eine vormontierte Baueinheit. An dem Behälter 2, genauer an der Krimphülse 3 des Behälters 2, ist ein geeigneter Austragkopf 1 montierbar. Die Krimphülse 3 weist zu diesem Zweck zwei im Wesentlichen plane, senkrecht zur Betätigungsrichtung I angeordnete Abstützflächen 30, 31 auf.

Zur Montage des in Fig. 1 dargestellten Austragkopfs 1 an dem Behälter 2 gemäß Fig. 2 umfasst der Austragskopf 1 ein Zwischenelement 10 mit einer Aufnahmeöffnung 11 für den Behälter 2.

In dem dargestellten Ausführungsbeispiel ist an der Aufnahmeöffnung 11 ist eine Rastgeometrie vorgesehen, welche ein Aufschnappen des Austragkopfs 1 auf die Krimphülse 3 des Behälters 2 ermöglicht. In dem dargestellten Ausführungsbeispiel umfasst die Rastgeometrie mehrere, insbesondere vier Rasthaken 12 oder Rastvorsprünge, wobei in Fig. 1 nur zwei Rasthaken 12 sichtbar sind. Die Rasthaken 12 weisen jeweils eine Formschräge 120 auf, welche ein Einführen des Behälters 2 entlang der Betätigungsrichtung I in das Zwischenelement 10 ermöglichen oder zumindest erleichtern. In einer Ausgestaltung sind Rastvorsprünge vorgesehen, welche zu der Betätigungsrichtung I im Wesentlichen senkrecht stehende Hakenanschläge aufweisen. In dem dargestellten Ausführungsbeispiel sind die Rasthaken 12 als Federarme gestaltet, welche die eingeführte Krimphülse 3 umgreifen und eine Relativbewegung des Behälters 2 und des Zwischenelements 10 nach einem Verrasten zum Entnehmen des Behälters 2 aus dem Zwischenelement 10 verhindern. Für ein sicheres Verrasten sind die Rasthaken 12 geeignet dimensioniert.

An dem Zwischenelement 10 sind weiter in den Innenraum des Zwischenelements 10 ragende Anschläge 13 vorgesehen, welche ebenfalls mit der Krimphülse 3 zusammenwirken und eine Einführbewegung des Behälters 2 in das Zwischenelement 10 begrenzen. Nach dem Einführen des Behälters 2 in das Zwischenelement 10 ist somit die Krimphülse 3 zwischen den Rasthaken 12 und den Anschlägen 13 des Zwischenelements 10 angeordnet und so mit dem Zwischenelement 10 verbunden. Ein Abstand zwischen den Rasthaken 12 und den Anschlägen 13 ist in den dargestellten Ausführungsbeispielen größer als eine Höhe H der Krimphülse 3, sodass die Krimphülse 3 unter Spiel mit dem Zwischenelement 10 verrastet wird.

Der in Fig. 1 dargestellte Austragkopf 1 umfasst weiter eine mit dem Zwischenelement 10 gekoppelte Betätigungshandhabe 14, welche in dem dargestellten Ausführungsbeispiel als Gehäuse des Austragkopfs 1 gestaltet ist.

An der Betätigungshandhabe 14 ist gegenüberliegend zu der Aufnahmeöffnung 11 für den Behälter 2 gemäß Fig. 2 eine Applikatorspitze 15 mit einer Austragöffnung 16 für das Medium vorgesehen. Die Austragöffnung 16 ist über einen Austragkanal 17 mit einem Auslass der in dem Behälter 2 angeordneten Pumpeinheit 4 verbindbar. Die Applikatorspitze 15 ist für einen Gebrauch geeignet gestaltet, beispielsweise als Nasenolive. Die Austragöffnung 16 ist bedarfsweise mittels einer nicht dargestellten Kappe verschließbar. In dem Austragkanal 17 ist ein Stift 7 vorgesehen, durch welchen die Pumpeinheit 4 betätigbar ist.

Die Betätigungshandhabe 14 ist für einen Austrag relativ zu dem Zwischenelement 10 in Betätigungsrichtung I verschieblich. Ein Verschieben erfolgt dabei entlang eines durch Anschläge begrenzten Verschiebewegs. Die Umkehrpunkte des Verschiebewegs werden als oberer und unterer Totpunkt bezeichnet, wobei bei Erreichen des oberen Totpunkts die Betätigungshandhabe 14 und das Zwischenelement 10 maximal auseinandergeschoben und bei Erreichen des unteren Totpunkts die Bauteile maximal ineinander geschoben sind.

Um eine undefinierte Lage der Betätigungshandhabe 14 relativ zu dem Zwischenelement 10 bei einer Montage des Austragkopfs 1 an dem Behälter 2 zu verhindern, sind Verbindungselemente vorgesehen, welche eine Haltekraft zwischen dem Zwischenelement 10 und der Betätigungshandhabe 14 bewirken, die einer Relativbewegung zwischen dem Zwischenelement 10 und der Betätigungshandhabe 14 in Betätigungsrichtung I entgegensteht.

In dem dargestellten Ausführungsbeispiel weist das Zwischenelement 10 an einem der Aufnahmeöffnung 11 gegenüberliegenden Rand einen umlaufenden, radial nach außen ragenden Rasthaken 100 auf, welcher als Verbindungselement dient. Der Rasthaken 100 greift in eine Nut, welche durch einen radial nach innen ragenden Rasthaken 141 und mehrere, vorzugsweise vier über den Umfang verteilte, in den Innenraum der Betätigungshandhabe 14 ragende Vorsprünge 142 an der Betätigungshandhabe 14 gebildet ist.

In anderen Ausgestaltungen sind mehrere, in Umfangsrichtung verteilte Rasthaken an dem Zwischenelement 10 und/oder der Betätigungshandhabe 14 vorgesehen. Dabei ist eine Größe der Rasthaken und/oder ein Abstand zwischen den Rasthaken vorzugsweise derart gewählt, dass ein Lösen der Verbindung durch Verdrehen nicht möglich ist. In anderen Ausgestaltungen sind zumindest die Rasthaken des Zwischenelements 10 und die Vorsprünge 142 so dimensioniert, dass die Verbindung durch Verdrehen lösbar ist. Alternativ sind in anderen Ausgestaltungen an einer Mantelfläche der Betätigungshandhabe 14 Durchbrüche vorgesehen, in welche entsprechende, über dem Umfang verteilte Rasthaken eingreifen.

Die Rasthaken 100, 141 und die Vorsprünge 142 weisen einander zugewandte Begrenzungsflächen auf, mittels welchen sie in dem in Fig. 1 dargestellten Zustand aneinander anliegen, sodass eine Haltekraft über die Begrenzungsflächen übertragen wird. Das Zwischenelement 10 und/oder die Betätigungshandhabe 14 sind aus einem elastischen Material, sodass durch Aufbringen einer Montagekraft in Betätigungsrichtung I, welche die Haltekraft übersteigt, der Rasthaken 100 die Vorsprünge 142 überfährt und somit eine axialfeste Verbindung zwischen dem Zwischenelement 10 und der Betätigungshandhabe 14 gelöst wird. Der Rasthaken 100 weist zu diesem Zweck an einer an dem Vorsprung 142 zugewandten Seite eine Formschräge auf. An der dem Rasthaken 141 zugewandten Seite des Rasthakens 100 verhindern im Wesentlichen senkrecht zu der Betätigungsrichtung I angeordnete Anschlagflächen, dass bei Aufbringen einer Kraft in entgegen gesetzter Richtung die Vorsprünge 142 durch den Rasthaken 100 wieder überfahren werden.

Die Vorsprünge 142 und der Rasthaken 100 dienen nach einer Montage auch zur Begrenzung des Verschiebewegs bei einem Austrag. Mittels der Verbindungselemente ist die Betätigungshandhabe 14 somit an dem Zwischenelement 10 in einem Bereich hinter dem oberen Totpunkt fixiert, sodass die Bauteile bei der Montage über den Bereich des Verschiebewegs auseinandergeschoben sind.

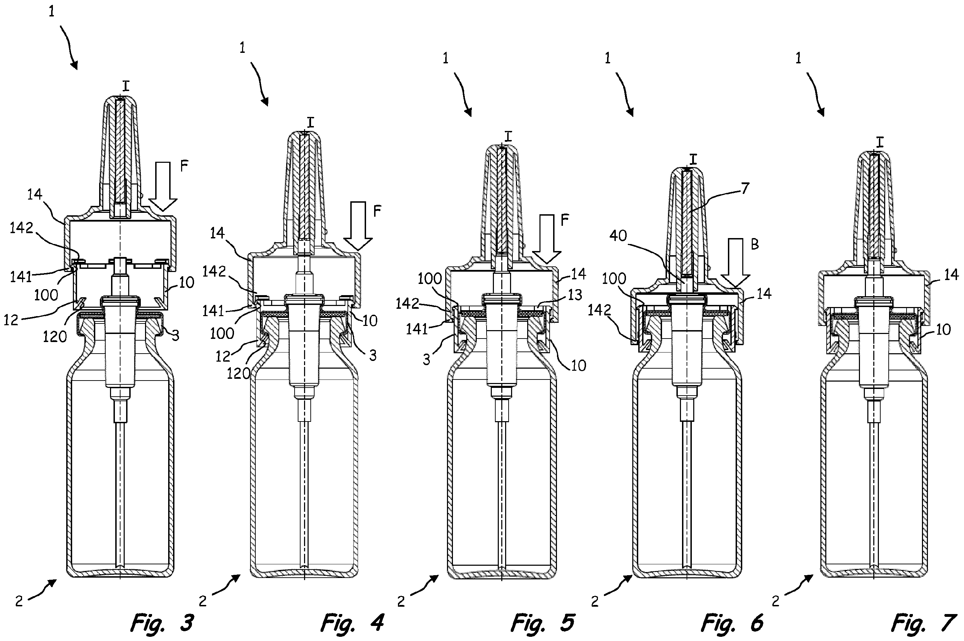

Die Fig. 3 bis 5 zeigen eine Montage des Austragkopfs gemäß Fig. 1 an einem Behälter 2 gemäß Fig. 2.

Wie in Fig. 3 erkennbar ist, werden der Austragkopf 1 und der Behälter 2 als vormontierte Baueinheiten bereitgestellt. Insbesondere ist beispielsweise vorgesehen, dass eine den Grundkörper 20 des Behälters 2 verschließende Krimphülse 3 mit einer davon aufgenommenen Pumpeinheit 4 nach einem Befüllen des Behälters 2 angebracht wird.

Der Austragkopf 1 und der Behälter 2 werden koaxial zueinander ausgerichtet und durch Aufbringen einer in Betätigungsrichtung I wirkenden Montagekraft F an der Betätigungshandhabe 14 aufeinander zu bewegt. In dem in Fig. 3 dargestellten ersten Montageschritt ist die Betätigungshandhabe 14 mittels der Verbindungselemente 100, 141, 142 an dem Zwischenelement 10 fixiert. Ein Kraftangriffspunkt der Montagekraft F ist lediglich beispielhaft dargestellt und durch den Fachmann geeignet wählbar. Beispielsweise wird in einer Ausgestaltung eine Montagekraft zum Befestigen des Austragkopfs 1 an dem Behälter 2 an dem Zwischenelement 10 aufgebracht.

Bei einer Bewegung des Austragkopfs 1 relativ zu dem Behälter 2 wird - wie schematisch in Fig. 4 dargestellt - das Zwischenelement 10 mit dem Behälter 2, genauer mit der Krimphülse 3 verrastet. In dem dargestellten Ausführungsbeispiel sind die Rasthaken 12 der Rastgeometrie als Federarme gestaltet, wobei an den Formschrägen 120 durch die eingeführte Krimphülse 3 ein Kraft aufgebracht wird, durch welche die Rasthaken 12 elastisch verformt werden. Die Formschrägen 120 an den Rasthaken 12 bewirken auch eine Zentrierung des Austragkopfs 1 relativ zu dem Behälter 2. Bei Wegfall der Kraft an den Formschrägen 120 federn die Rasthaken 12 in die ursprüngliche Form hinter den eingeführten Krimphülse 3 zurück. Nach einem Verrasten verhindern die mit der Krimphülse 3 zusammenwirkenden Rasthaken 12 eine Relativbewegung des Behälters 2 und des Zwischenelements 10 zum Entnehmen des Behälters 2 aus dem Zwischenelement 10. Die Rastgeometrie umfassend die Rasthaken 12 zum Befestigen des Zwischenelements 10 an der Krimphülse 3 und die Verbindungselemente 100, 141, 142 sind dabei derart aufeinander abgestimmt, dass eine durch die Verbindungselemente 100, 141, 142 aufgebrachte Haltekraft größer ist als eine zum Verrasten aufzubringende Kraft. Bei Aufbringen der Montagekraft F kommt es daher zunächst zu einem Aufschnappen des Zwischenelements 10 auf die Krimphülse 3.

Eine Bewegung des Zwischenelements 10 relativ zu der Krimphülse 3 aufgrund der einwirkenden Montagekraft F ist - wie in Fig. 5 dargestellt - durch die Anschläge 13 begrenzt. Die Anschläge 13 stoppen somit eine Bewegung des Zwischenelements 10 aufgrund der aufgebrachten Montagekraft F. Durch Aufbringen einer weiteren in Betätigungsrichtung I wirkenden Montagekraft, wirken nun auf die Verbindungselemente 100, 141, 142 Kräfte, welche die Haltekraft der Verbindungselemente 100, 141, 142 übersteigen. Wie in Fig. 5 dargestellt, führt dies dazu, dass eine Verbindung zwischen dem Zwischenelement 10 und der Betätigungshandhabe 14 gelöst wird und der Rasthaken 100 die Vorsprünge 141 überfährt.

Fig. 6 und 7 zeigen schematisch eine Betätigung des montierten Austragkopfs 1 gemäß Fig. 1 zum Austragen eines Mediums. Wie in Fig. 6 dargestellt, ist für eine Betätigung eine Kraft in Betätigungsrichtung I an der Betätigungshandhabe 14 aufzubringen, mittels welcher die Betätigungshandhabe 14 mit dem Stift 7 relativ zu dem Behälter 2 verschoben wird. Aufgrund der Bewegung wird der Kolben 40 mittels des Stifts 7 entgegen der Kraft der Rückstellfeder verschoben. Ein Verschiebeweg der Betätigungshandhabe 14 ist in dem dargestellten Ausführungsbeispiel durch den Verstellweg des Kolbens 40 begrenzt. Die Rasthaken 100 schlagen daher in dem dargestellten Zustand nicht an einer von dem Behälter 2 abgewandten Begrenzungsfläche der Betätigungshandhabe 14 an, sondern sind wie dargestellt zu dieser beanstandet.

Bei Wegfall der Betätigungskraft B werden die Betätigungshandhabe 14 und das Zwischenelement 10 durch die Kraft der Rückstellfeder der Pumpeinheit 4 auseinander geschoben. Ein Verschiebeweg ist dabei durch die Verbindungselemente 100, 142 begrenzt, wobei der Rasthaken 100 an einer Seite der Vorsprünge 142 anschlägt, welche von dem Rasthaken 142 am Rand der Betätigungshandhabe 14 abgewandt ist und aufgrund der Gestaltung des Rasthakens 100 des Zwischenelements 10 und der Vorsprünge 142 verhindert wird, dass der Rasthaken 100 die Vorsprüngen 142 überfährt.

## Patentansprüche

1. Austragkopf zum Austragen eines flüssigen oder pastösen Mediums, welcher an einem das Medium bevorratenden Behälter (2) befestigbar ist, umfassend ein Zwischenelement (10) mit einer Aufnahmeöffnung (11) für den Behälter (2) und eine in einer Betätigungsrichtung (I) verschieblich mit dem Zwischenelement (10) gekoppelte Betätigungshandhabe (14), wobei das Zwischenelement (10) und/oder die Betätigungshandhabe (14) mindestens ein lösbares Verbindungselement (100, 141, 142) aufweisen, das eine Haltekraft zwischen dem Zwischenelement (10) und der Betätigungshandhabe (14) bewirkt, welche einer Relativbewegung zwischen dem Zwischenelement (10) und der Betätigungshandhabe (14) in Betätigungsrichtung (I) entgegensteht, und wobei die Aufnahmeöffnung (11) eine Rastgeometrie zur Befestigung an dem Behälter (2) aufweist, **dadurch gekennzeichnet, dass** die Rastgeometrie so gestaltet ist, dass das Zwischenelement (10) durch eine Bewegung in Betätigungsrichtung auf den Behälter (2) aufschnappbar und/oder aufprellbar ist und eine zum Aufschnappen und/oder Aufprellen des Zwischenelements (10) auf den Behälter (2) aufzubringende Kraft kleiner ist als die durch das mindestens eine Verbindungselement (100, 141, 142) aufgebrachte Haltekraft zwischen dem Zwischenelement (10) und der Betätigungshandhabe (14) in Betätigungsrichtung (I).

2. Austragkopf nach Anspruch 1, **dadurch gekennzeichnet, dass** das Zwischenelement (10) und die Betätigungshandhabe (14) nach Lösen des mindestens einen Verbindungselements (100, 141, 142) innerhalb eines Verschiebewegs in Betätigungsrichtung (I) verschieblich miteinander gekoppelt sind und das mindestens eine Verbindungselement (100, 141, 142) außerhalb des Verschiebewegs angeordnet ist.

3. Austragkopf nach Anspruch 2, **dadurch gekennzeichnet, dass** an der Aufnahmeöffnung (11) mindestens ein Anschlag (13) vorgesehen ist, mittels welchem eine Bewegung des Zwischenelements (10) relativ zu dem Behälter (2) beim Aufschnappen und/oder Aufprellen in Betätigungsrichtung (I) begrenzt ist.

4. Spender für ein flüssiges oder pastöses Medium, mit einem das Medium bevorratenden Behälter (2) und einem an dem Behälter (2) befestigten Austragkopf (1) nach einem der Ansprüche 1 bis 3.

5. Spender nach Anspruch 4, **dadurch gekennzeichnet, dass** an einer Öffnung (20) des Behälters (2) eine Krimphülse (3) angeordnet ist.

6. Verfahren zum Befestigen eines Austragkopfs (1) zum Austragen eines flüssigen oder pastösen Mediums an einem das Medium bevorratenden Behälter (2), wobei der Austragkopf (1) ein Zwischenelement (10) mit einer Aufnahmeöffnung (11) für den Behälter (2) und eine in eine Betätigungsrichtung (I) verschieblich mit dem Zwischenelement (10) gekoppelte Betätigungshandhabe (14) umfasst und wobei die Aufnahmeöffnung (11) eine Rastgeometrie zur Befestigung an dem Behälter (2) aufweist,
umfassend den Schritt Aufschnappen und/oder Aufprellen des Zwischenelements (10) auf den Behälter (2) durch Bewegung der mit dem Zwischenelement (10) gekoppelten Betätigungshandhabe in Betätigungsrichtung (I), wobei
- beim Aufschnappen und/oder Aufprellen des Zwischenelements (10) auf den Behälter (2) eine Relativbewegung zwischen dem Zwischenelement (10) und der Betätigungshandhabe (14) in Betätigungsrichtung (I) durch mindestens ein Verbindungselement (100, 141, 142), das eine Haltekraft in Betätigungsrichtung (I) bewirkt, gesperrt wird,
- nach einem Aufschnappen und/oder Aufprellen des Zwischenelements (10) auf den Behälter (2) das mindestens eine Verbindungselement (100, 141, 142) gelöst wird, und
- das Zwischenelement (10) auf den Behälter (2) aufgeschnappt und/oder aufgeprellt wird, bevor auf das mindestens eine Verbindungselement (100, 141, 142) eine Kraft wirkt, welche die durch das mindestens eine Verbindungselement (100, 141, 142) aufgebrachte Haltekraft zwischen dem Zwischenelement (10) und der Betätigungshandhabe (14) in Betätigungsrichtung (I) übersteigt.

7. Verfahren nach Anspruch 6, **dadurch gekennzeichnet, dass** eine Bewegung des Zwischenelement (10) relativ zu dem Behälter (2) beim Aufschnappen und/oder Aufprellen durch mindestens einen an der Aufnahmeöffnung (11) angeordneten Anschlag (13) begrenzt wird, wobei durch eine weitere Bewegung der Betätigungshandhabe (14) in Betätigungsrichtung (I) auf das mindestens eine Verbindungselement in Betätigungsrichtung (I) eine die Haltekraft übersteigende Kraft aufgebracht wird, sodass das mindestens eine Verbindungselement (100, 141, 142) gelöst wird.

8. Verfahren nach Anspruch 6 oder 7, **dadurch gekennzeichnet, dass** an einer Öffnung des Behälters (2) eine Ventileinheit und/oder eine Pumpeinheit mittels einer Krimphülse vormontiert wird.

## Claims

1. Discharge head for discharging a liquid or pasty medium, which discharge head is fastenable to a container (2) storing the medium, comprising an intermediate element (10) with a receiving opening (11) for the container (2) and an actuating handle (14) which is coupled displaceably in an actuating direction (I) to the intermediate element (10), wherein the intermediate element (10) and/or the actuating handle (14) have/has at least one releasable connecting element (100, 141, 142) which brings about a retaining force between the intermediate element (10) and the actuating handle (14), which retaining force is opposed to a relative movement between the intermediate element (10) and the actuating handle (14) in the actuating direction (I), and wherein the receiving opening (11) has a latching geometry for the fastening to the container (2), **characterized in that** the latching geometry is designed in such a manner that the intermediate element (10) is snappable and/or pushable onto the container (2) by a movement in the actuating direction, and a force to be applied for snapping and/or pushing the intermediate element (10) onto the container (2) is smaller than the retaining force, which is applied by the at least one connecting element (100, 141, 142), between the intermediate element (10) and the actuating handle (14) in the actuating direction (I).

2. Discharge head according to Claim 1, **characterized in that**, after release of the at least one connecting element (100, 141, 142), the intermediate element (10) and the actuating handle (14) are coupled displaceably to each other within a displacement distance in the actuating direction (I), and the at least one connecting element (100, 141, 142) is arranged outside the displacement distance.

3. Discharge head according to Claim 2, **characterized in that** at least one stop (13) is provided on the receiving opening (11), said stop being used to limit a movement of the intermediate element (10) relative to the container (2) in the actuating direction (I) during the snapping-on and/or pushing-on operation.

4. Dispenser for a liquid or pasty medium, with a container (2) storing the medium and with a discharge head (1), which is fastened to the container (2), according to one of Claims 1 to 3.

5. Dispenser according to Claim 4, **characterized in that** a crimped sleeve (3) is arranged on an opening (20) of the container (2).

6. Method for fastening a discharge head (1), for discharging a liquid or pasty medium, to a container (2) storing the medium, wherein the discharge head (1) comprises an intermediate element (10) with a receiving opening (11) for the container (2) and an actuating handle (14) which is coupled displaceably in an actuating direction (I) to the intermediate element (10), and wherein the receiving opening (11) has a latching geometry for the fastening to the container (2), comprising the step of snapping and/or pushing the intermediate element (10) onto the container (2) by movement of the actuating handle, which is coupled to the intermediate element (10), in an actuating direction (I), wherein
- during the snapping and/or pushing of the intermediate element (10) onto the container (2), a relative movement between the intermediate element (10) and the actuating handle (14) in the actuating direction (I) is blocked by at least one connecting element (100, 141, 142), which brings about a retaining force in the actuating direction (I),
- after the intermediate element (10) is snapped and/or pushed onto the container (2), the at least one connecting element (100, 141, 142) is released, and
- the intermediate element (10) is snapped and/or pushed onto the container (2) before a force acts on the at least one connecting element (100, 141, 142), said force exceeding the retaining force, which is applied by the at least one connecting element (100, 141, 142), between the intermediate element (10) and the actuating handle (14) in the actuating direction (I).

7. Method according to Claim 6, **characterized in that** a movement of the intermediate element (10) relative to the container (2) during the snapping-on and/or pushing-on operation is limited by at least one stop (13) arranged on the receiving opening (11), wherein, by a further movement of the actuating handle (14) in the actuating direction (I), a force exceeding the retaining force is applied to the at least one connecting element in the actuating direction (I), and therefore the at least one connecting element (100, 141, 142) is released.

8. Method according to Claim 6 or 7, **characterized in that** a valve unit and/or a pump unit is prefitted on an opening of the container (2) by means of a crimped sleeve.

## Revendications

1. Tête de distribution pour distribuer un milieu liquide ou pâteux, qui peut être fixée à un récipient (2) stockant le milieu, comprenant un élément intermédiaire (10) avec une ouverture de réception (11) pour le récipient (2) et une manette d'actionnement (14) accouplée à l'élément intermédiaire (10) de manière déplaçable dans une direction d'actionnement (I), l'élément intermédiaire (10) et/ou la manette d'actionnement (14) présentant au moins un élément de connexion amovible (100, 141, 142) qui exerce une force de retenue entre l'élément intermédiaire (10) et la manette d'actionnement (14), laquelle s'oppose à un mouvement relatif entre l'élément intermédiaire (10) et la manette d'actionnement (14) dans la direction d'actionnement (I), et l'ouverture de réception (11) présentant une géométrie d'encliquetage pour la fixation au récipient (2), **caractérisée en ce que** la géométrie d'encliquetage est configurée de telle sorte que l'élément intermédiaire (10) puisse être enclipsé et/ou encliqueté sur le récipient (2) par un déplacement la direction d'actionnement et une force devant être appliquée pour l'enclipsage et/ou l'encliquetage de l'élément intermédiaire (10) sur le récipient (2) est inférieure à la force de retenue appliquée par l'au moins un élément de connexion (100, 141, 142) entre l'élément intermédiaire (10) et la manette d'actionnement (14) dans la direction d' actionnement (I).

2. Tête de distribution selon la revendication 1, **caractérisée en ce que** l'élément intermédiaire (10) et la manette d'actionnement (14), après le desserrage de l'au moins un élément de connexion (100, 141, 142), sont accouplés l'un à l'autre de manière déplaçable à l'intérieur d'une course de déplacement dans la direction d'actionnement (I) et l'au moins un élément de connexion (100, 141, 142) est disposé à l'extérieur de la course de déplacement.

3. Tête de distribution selon la revendication 2, **caractérisée en ce qu'**au moins une butée (13) est prévue au niveau de l'ouverture de réception (11), au moyen de laquelle un déplacement de l'élément intermédiaire (10) par rapport au récipient (2) lors de l'enclipsage et/ou de l'encliquetage est limité dans la direction d'actionnement (I).

4. Distributeur pour un milieu liquide ou pâteux, comprenant un récipient de stockage de milieu (2) et une tête de distribution (1) fixée au récipient (2), selon l'une quelconque des revendications 1 à 3.

5. Distributeur selon la revendication 4, **caractérisé en ce qu'**une douille de sertissage (3) est disposée au niveau d'une ouverture (20) du récipient (2).

6. Procédé pour la fixation d'une tête de distribution (1) pour distribuer un milieu liquide ou pâteux à partir d'un récipient (2) stockant le milieu, la tête de distribution (1) comprenant un élément intermédiaire (10) avec une ouverture de réception (11) pour le récipient (2) et une manette d'actionnement (14) accouplée à l'élément intermédiaire (10) de manière déplaçable dans une direction d'actionnement (I) et l'ouverture de réception (11) présentant une géométrie d'encliquetage pour la fixation au récipient (2), comprenant l'étape consistant à enclipser et/ou encliqueter l'élément intermédiaire (10) sur le récipient (2) par déplacement dans la direction d'actionnement (I) de la manette d'actionnement accouplée à l'élément intermédiaire (10),
- un mouvement relatif entre l'élément intermédiaire (10) et la manette d'actionnement (14) dans la direction d'actionnement (I), lors de l'enclipsage et/ou de l'encliquetage de l'élément intermédiaire (10) sur le récipient (2), étant bloqué par au moins un élément de connexion (100, 141, 142) qui provoque une force de retenue dans la direction d'actionnement (I),
- après un enclipsage et/ou encliquetage de l'élément intermédiaire (10) sur le récipient (2), l'au moins un élément de connexion (100, 141, 142) est desserré, et
- l'élément intermédiaire (10) est enclipsé et/ou encliqueté sur le récipient (2) avant qu'une force n'agisse sur l'au moins un élément de connexion (100, 141, 142), laquelle dépasse la force de retenue appliquée par l'au moins un élément de connexion (100, 141, 142) entre l'élément intermédiaire (10) et la manette d'actionnement (14) dans la direction d' actionnement (I).

7. Procédé selon la revendication 6, **caractérisé en ce qu'**un déplacement de l'élément intermédiaire (10) par rapport au récipient (2) lors de l'enclipsage et/ou de l'encliquetage est limité par au moins une butée (13) disposée au niveau de l'ouverture de réception (11), un déplacement supplémentaire de la manette d'actionnement (14) dans la direction d'actionnement (I) vers l'au moins un élément de connexion générant l'application d'une force dépassant la force de retenue dans la direction d'actionnement (I), de sorte que l'au moins un élément de connexion (100, 141, 142) soit desserré.

8. Procédé selon la revendication 6 ou 7, **caractérisé en ce qu'**une unité de soupape et/ou une unité de pompe est prémontée au moyen d'une douille de sertissage au niveau d'une ouverture du récipient (2).
